# EUROPEAN PATENT APPLICATION

(11) **EP 3 146 890 A2**
(43) Date of publication of application: **29.03.2017**
(21) Application number: 16001902.2
(22) Date of filing: 12.02.2009
(51) Int. Cl.: A61B 1/06

(54) **RADIAL SCANNER IMAGING SYSTEM**

(30) Priority: 12.02.2008 US 28102 P; 21.02.2008 US 30453 P
(62) Divisional of application: 09711510.9
(71) Applicant: Innurvation, Inc., Columbia, MD 21046-1169 (US); Bandy, William R., Gambrills, MD 21054 (US); Jamieson, Brian Glenn, Severna Park, MD 21146 (US); Powell, Kevin J., Annapolis, MD 21403 (US); Salsman, Kenneth E., Pleasanton, CA 94566 (US); Schober, Robert Charles, Huntington Beach, CA 92646 (US); Weitzner, John, Coto De Caza, CA 92679 (US); Arneson, Michael R., Finksburg, MD 21048 (US); Grunnet-Jepsen, Anders, San Jose, CA 95139 (US)
(72) Inventor: Bandy, William Robert, Gambrills, MD 21054 (US); Jamieson, Brian, Severna Park, MD 21146 (US); Powell, Kevin James, Annapolis, MD 21403 (US); Salsman, Kenneth Edward, Pleasanton, CA 94566 (US); Schober, Robert Charles, Huntington Beach, CA 92646 (US); Weitzner, John, Coto De Caza, CA 92679 (US); Arneson, Michael, R., Finksburg, MD 21048 (US); Grunnet-Jepsen, Anders, San Jose, CA 95139 (US)
(74) Representative: Jehle, Volker Armin

(57) **Abstract**

According to an aspect, an ingestible scanning device is provided, comprising: an ingestible capsule housing having a transparent window; a photo-sensing array located within the capsule housing; a mirror located within the housing and oriented to direct an image from a surface outside the transparent window to the photo-sensing array; and a light source for illuminating the surface outside the transparent window.

## Description

### CROSS-REFERENCE TO RELATED APPLICATIONS

The present application claims the benefit of U.S. Provisional Patent Appl. No. 61/028,102, filed February 12, 2008, and U.S. Provisional Patent Appl. No. 61/030,453, filed February 21, 2008, each of which is incorporated by reference herein in its entirety.

### BACKGROUND

### Field

Embodiments of the present invention relate to optical scanners, specifically optical scanners located on an ingestible endoscopic device and imaging systems for use inside tubular structures of the body, such as intestines and veins.

### Related Art

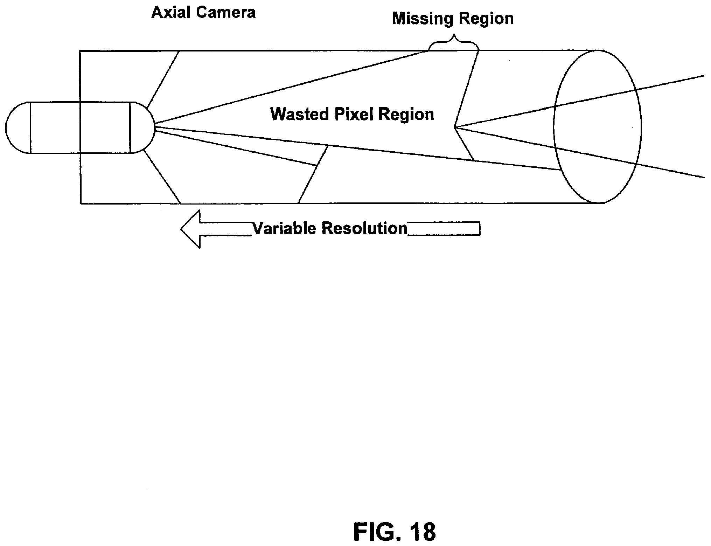

Modem electronic imaging systems typically utilize a two dimensional focal plane array (FPA). For example, borescopes, endoscopes, and similar tools used to inspect or image tubular structures have relied on classic camera optical system designs, where a lens is used to capture a full 2D image that is then focused down an optical fiber or projected onto an electronic image capture device such as a CCD or CMOS imaging FPA. This requires the optical system to have a very wide angle lens so as to capture the image of the tubular wall closest to the optics, or to provide a flexible or mirror deflective system so that the operator can move the field of view into the realm of 90 degrees from the axis of the tubular structure being examined. Additionally, this requires the use of a single optical lens element with a stop that is circularly symmetrical in two dimensions to focus the image onto all of the imaging FPA. Currently, small imaging systems such as those used in camera pills for biomedical cameras, as well as for security and industrial applications, are limited in resolution by the pixels in their FPA chip. As a result, the current camera pills for gastric intestinal (also referred to herein as endoscopic) examination have a relatively low resolution of, for example, 300 x 300 pixels. This results in images that provide extremely limited details and cannot be enlarged significantly for improved diagnostic analysis. As a result of this design approach, several performance issues occur. A conventional camera having these performance issues is illustrated in FIG. 18.

For example, these systems are impaired with the orientation of the array such that the field of view of the camera pill is continuous and fixed. For example, a camera pill may only look down the central axis of the pill and GI tract thereby presenting the center of the field of view as having very little to no useful image data. Additionally, these camera pills typically require extremely wide angle "fish-eye" lenses to image the tissue along the intestinal wall with the result that the images are distorted and non-uniformly illuminated.

Likewise, in tubular environments, the highest resolution can be achieved while imaging surfaces that are closest to the lens. Wide angle lenses provide the ability to see the circumference of the tubular structure, but suffer in image quality as the light from the surface undergoes more radical deflection. This means that a lens that is looking down a tube with the center of the tube and the center of the lens in alignment will provide an image of the closest surfaces in the region of the lens where there is more optical distortion. Pixels in this peripheral region of the lens typically show significant coma or comet-like shapes, and lose their true geometric format. In a performance paradox, the center of the field of view where there is the least optical distortion is where the angle of the surface and the distance to the surface is the worst, and provides the least useful image performance. For example, use of a fish-eye lens generates images where the information of the condition of the inside wall of the GI tract is only contained in a ring of pixels at the peripheral region of the image. In this situation the center region of the image shows a view down the length of the intestine and contains little or no useful information. This results in images where the most important data is presented on a small fraction of the pixels in a focal plane imaging array such as a CCD or CMOS imager. This reduction in the effective resolution is exacerbated by the presentation of the image in a distorted circular appearance with the most outer edge visible being brightly lit and close to the imaging array while the rest of the intestinal wall surface is progressively further away from the camera and progressively dimmer in illumination.

Additionally, the images of interest to a physician are images of the intestinal wall, not the forward-looking view down the GI tract. The ability to scan the intestinal walls is thus preferred over traditional camera pill imaging. The ability for the capsule to image the entire tubular wall and the size and electrical connections required for the focal plane array define that the image array be set so that the imaging surface is perpendicular to the axis of the pill.

Another performance issue is variable resolution. A camera looking down the axis of a tube will see the highest resolution of the surface at the closest point of focus to the lens. However, the angle of the surface to the axis of the camera causes the area seen by any one pixel in the imaging system to increase rapidly as the location of the surface being imaged moves away from this closest point. This steep angular orientation produces a drop in resolution and a distorted perspective of the surface features that are visible.

Another performance issue is variable illumination. As the tubular surface being examined by a co-axial camera moves further from the camera, the steep angle to the surface further decreases the amount of effective illumination of the surface. This causes a rapid drop in relative brightness and produces a highly non-linear illumination within the field of view. To maximize the amount of surface being viewed in each image, the closest surfaces are typically overexposed, further reducing the quality of the image.

Yet another performance issue is loss of useful pixels. Cameras use either film or focal plane arrays designed to capture images in a two-dimensional planar manner. As a result, images taken down the axis of a tubular structure are akin to the proverbial round peg in a square hole. The image of a tube taken with a wide angle lens that is in axial alignment with the tube shows a circular image area where the region between the circular area and the rectangular area of the photo sensing imager remains unused. This typically results in a loss of about 20% of the resolution. Further, due to the extreme angle to the surface and resultant poor lighting, the surface area in the center of the field of view is of exceedingly low value. This leaves only a ring of pixels in each image where the pixel value is high enough to provide the user with viable information.

Another performance issue is the requirement for dynamic range. The geometry issues with axial imaging of a tubular structure involve the variance in intensity of illumination from the distant surface in the center of the field of view to the nearby surface area at the periphery of the field of view. This generates a very wide range of illumination intensities, which increases the difficulty in producing an image with a maximum of valuable data. When the mid-range tissue is illuminated to the correct level for maximum contrast, the tissue near the camera will be overexposed, and the tissue in the center of the field of view will be underexposed. This situation limits the ability of the system to collect optimized images of the entire visible surface region. As such, systems taking images at a time interval while moving through the tubular structure produce data sets where significant regions are either not in the image or are not imaged at an optimal illumination level.

Obtaining the required depth of focus is also difficult. Due to the long range of distances within the field of view of an axially oriented camera, the system must either take multiple images at various focus settings, utilize a high stop setting (that is, minimize the aperture of the lens) or use a lens which has a long focal length relative to its diameter. These options all allow the ability to generate a final image with a majority of the image in focus. However, there are significant trade-offs in these options with very negative impacts. Use of multiple exposures at multiple focus settings can allow for a composite image to be generated with essentially the entire tubular surface in focus. This technique is used in microphotography of biological tissues to allow vascular and cellular structures with large, three-dimensional structures to be captured fully in focus. The capture of multiple images takes significant time and power, and generates very large data files for imaging a single region of the surface. Use of an aperture stop or long focus optical system both result in large depths of focus, but they also are very inefficient in utilization of light. Long focal length lenses also do not have the ability to focus on nearby surfaces, thereby limiting the ability of this approach to produce good images of the region where a wide angle lens provides its best performance and the surface is at the best location for imaging.

Another performance issue is an inability to provide an appropriate F-stop for the entire image. As mentioned previously, optical systems may be equipped with an optical stop or restriction which reduces the relative aperture of the lens. This results in an increase of the focal range, but at the expense of the optical efficiency of the system. By reducing the functional size of the lens, the light collection ability of the optics is reduced, resulting in the need for higher levels of illumination and more power needed to drive the imaging system. This has a very detrimental impact on any system being designed for portable or battery operation.

Steerable or restricted field of view systems also suffer from performance issues. Endoscopes are typically built with flexible fiber-optic heads that allow the user to bend the end of the scope and look at the sidewall of the tubular structure. Likewise, bore scopes can be equipped with 90 degree turning mirrors so that the unit can be used to view the side wall of the tubular structure it is inserted into. When these units are operated in this manner, they lose the ability to see the entire circumference of the tube. This restriction of the field of view requires the operator to keep track of parts of the wall surface that have been viewed. It also significantly increases the amount of images and time required to view the surface.

### SUMMARY

A radial scanner may be used to image the interior of a tube, such as an intestine or vasculature of an animal. Radial scanners can include optical systems capable of utilizing one or more photo sensors and a mechanical or electro-mechanical scanning optical element. This creates a line scan or a solid state array of photo sensors arranged in a format such that light from the subject can pass through the optics, which collects it in a circular format and onto the sensor array. In an embodiment, these optical elements include a standard camera imager and a lens axially aligned to a cylindrically-symmetrical mirror such as a cone mirror, which directs the light from the subject at a 90 degree deflection from the target surface to the lens. The lens focuses the image onto a ring of photo sensitive pixels fabricated on a semiconductor substrate.

In an embodiment, a ring array of prisms, lenses, and/or multi-faceted or conical mirrors may be used which capture the light from the target surface and direct it in at an angle from incidence to a second optical element. The second optical element includes image-forming elements which direct the light onto a pattern of photo sensitive pixels to produce an electronic image of the scan region. These multi-element optical arrays can be fabricated from single material structures, providing the ability to keep them in position. By utilizing alignment pins and spacers, the optical system may be easily assembled and aligned, with each element in the array kept at its appropriate location for optimal and uniform performance.

In another embodiment, fiber optical elements may be used to collect the light from the target surface through a lens element in the tip. Each element may then be directed to a pixel array to provide an image of the radial scan region.

In an embodiment, an ingestible scanning device includes a capsule housing having a transparent window and sized so as to be ingestible, a photo-sensing array located within the capsule housing, a mirror located within the housing and oriented to direct an image from a surface outside the transparent window to the photo-sensing array, and a light source for illuminating the surface outside the transparent window.

According to an aspect, a radial scanner is provided configured to image the interior of a tube, comprising: a housing having a transparent window; a photo-sensing array; a mirror located within the housing and oriented to direct an image around a circumference of an interior surface of the tube outside the transparent window to the photo-sensing array; and a light source configured to illuminate the interior surface of the tube, wherein the photo-sensing array is configured to receive the image around the circumference as a circular line scan.

Advantageously, the mirror is a cylindrically-symmetrical reflective element or is an element combining reflective and refractive surfaces or is a radial set of prisms oriented such that reflective hypotenuse surfaces of the prisms direct images from the interior surface of the tube to the photo-sensing array.

Advantageously, the mirror has a hollowed-out conical shape.

Advantageously, the photo-sensing array comprises a ring of photo-sensing pixels.

Advantageously, the housing is an ingestible capsule and/or is tethered to an external unit configured to be located outside the tube via an optical fiber.

Advantageously, the photo-sensing array is located within the external unit.

According to an aspect, a medical catheter is provided comprising: a fiber optic cable; an intra-cavity unit coupled to a distal end of the fiber optic cable; and an external unit coupled to a proximal end of the fiber optic cable, wherein the external unit comprises: a photo-sensing array configured to receive a scanned image, and wherein the intra-cavity unit comprises: a housing coupled to the distal end of the fiber optic cable, the housing having a transparent window; a mirror located within the housing and oriented to direct an image around a circumference of an interior surface of a tubular structure outside the transparent window to the photo-sensing array via the fiber optic cable.

Advantageously, the intra-cavity unit further comprises a lens configured to direct the image from the mirror into the fiber optic cable.

Advantageously, the mirror has a conical shape and/or is a radial set of prisms oriented such that reflective hypotenuse surfaces of the prisms direct images from the interior surface of the tube to the photo-sensing array.

According to an aspect, an ingestible scanning device is provided, comprising: an ingestible capsule housing having a transparent window; a photo-sensing array located within the capsule housing; a mirror located within the housing and oriented to direct an image from a surface outside the transparent window to the photo-sensing array; and a light source for illuminating the surface outside the transparent window.

Advantageously, the mirror is rotatable and/or is a cylindrically symmetrical reflective element or is an element combining reflective and refractive surfaces.

Advantageously, the ingestible scanning device further comprises a cylindrical lens between the photo-sensing array and the mirror, the cylindrical lens being oriented to direct light from the mirror onto the photo-sensing array.

Advantageously, the ingestible scanning device further comprises a toroidal lens located around the photo-sensing array, the toroidal lens oriented to direct light from the light source onto the mirror.

Advantageously, the light source is located within the capsule housing or is attached to an outer surface of the capsule housing.

Advantageously, the light source is a light emitting diode or is a light distribution ring.

Further features and advantages of the invention, as well as the structure and operation of various embodiments of the invention, are described in detail below with reference to the accompanying drawings. It is noted that the invention is not limited to the specific embodiments described herein. Such embodiments are presented herein for illustrative purposes only. Additional embodiments will be apparent to persons skilled in the relevant art(s) based on the teachings contained herein.

### BRIEF DESCRIPTION OF THE DRAWINGS/FIGURES

The accompanying drawings, which are incorporated herein and form part of the specification, illustrate the present invention and, together with the description, further serve to explain the principles of the invention and to enable a person skilled in the relevant art(s) to make and use the invention.
FIG. 1 is an illustration of a radial scanner system according to an embodiment of the present invention.
FIG. 2 is an exemplary image having distortion caused by a cone-shaped optical element.
FIG. 3 is an exemplary result of processing performed on the image of FIG. 2.
FIG. 4 is an illustration of a radial scanner system according to another embodiment of the present invention.
FIG. 5 is an exemplary image of text.
FIG. 6 is an exemplary distorted image obtained according to an embodiment of the present invention.
FIG. 7 is an exemplary image with distortion removed according to an embodiment of the present invention.
FIG. 8 is an illustration of a radial scanner system having an illuminator according to an embodiment of the present invention.
FIG. 9 is an illustration of a radial scanner system having an illuminator according to another embodiment of the present invention.
FIG. 10 is an illustration of a radial scanner system according to another embodiment of the present invention.
FIG. 11 is an illustration of a radial scanner system according to another embodiment of the present invention.
FIG. 12 illustrates an ingestible capsule according to an embodiment of the present invention.
FIG. 13 illustrates an ingestible capsule according to another embodiment of the present invention.
FIG. 14 illustrates a tethered device according to an embodiment of the present invention.
FIG. 15 illustrates a combination camera and radial scanner system according to an embodiment of the present invention.
FIG. 16 is a flowchart of a method for obtaining a high-resolution image according to an embodiment of the present invention.
FIG. 17 illustrates an exemplary image as viewed by a radial scanning system according to an embodiment of the present invention.
FIG. 18 illustrates a conventional axial camera.
Figure 19 illustrates an ingestible scanner according to an embodiment of the present invention.
Figures 20A and 20B illustrate a lighting system for use in an ingestible scanner, according to an embodiment of the present invention.
Figure 21 illustrates an exemplary endcap of an ingestible scanner, according to an embodiment of the present invention.
Figure 22 illustrates additional exemplary endcaps of an ingestible scanner, according to an embodiment of the present invention.
Figure 23 illustrates a cross-section of an ingestible scanner according to an embodiment of the present invention.
Figure 24 illustrates a cross-section of another ingestible scanner according to an embodiment of the present invention.
Figure 25 illustrates a cross-section of another ingestible scanner according to an embodiment of the present invention.
Figure 26 illustrates exemplary scribes used in an embodiment of the present invention.
Figure 27A is a cross-section of a photodiode.
Figure 27B is a top-down view of the photodiode of Figure 27A.
Figures 28A and 28B illustrate construction of a back-lit scanner according to an embodiment of the present invention.
Figure 28C is a top-down view of a photosensor array using the scanner of Figures 28A and 28B.
Figure 29 illustrates an exemplary reflective element according to an embodiment of the present invention.
Figure 30 illustrates an exemplary reflective and refractive element according to an embodiment of the present invention.

The features and advantages of the present invention will become more apparent from the detailed description set forth below when taken in conjunction with the drawings, in which like reference characters identify corresponding elements throughout. In the drawings, like reference numbers generally indicate identical, functionally similar, and/or structurally similar elements.

### DETAILED DESCRIPTION

While specific configurations and arrangements are discussed, it should be understood that this is done for illustrative purposes only. A person skilled in the pertinent art will recognize that other configurations and arrangements can be used without departing from the spirit and scope of the present invention. It will be apparent to a person skilled in the pertinent art that this invention can also be employed in a variety of other applications.

It is noted that references in the specification to "one embodiment", "an embodiment", "an example embodiment", etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect such feature, structure, or characteristic in connection with other embodiments whether or not explicitly described.

### I. Radial Scanning System

A side-scanning optical system can provide improvements over conventional axial camera systems when used in tubular environments. In particular, scans of the interior surface of a tube, such as an intestine, vasculature, urinary tract, reproductive tract, lung, nasal or internal ear cavity, or any other cavity of an animal, can provide high-resolution images of the interior of the tube in an easily-examined format without a need for a surgical extraction (e.g., biopsy) of the tissue. For instance, directly scanning the surface of an intestine rather than viewing the intestine down the intestinal path allows a doctor to examine the intestine as if it has been cut apart and laid flat as a familiar viewpoint but without the need of a biopsy under microscope, rather than current state of the art requiring the doctor to determine surface features from a non-optimal viewpoint.

Embodiments of the present invention afford a doctor or medical professional to be able to view a single static display of the image of a full surface scan of a tubular structure or cavity. Current state of the art displays are in a bouncy, confusing format of a series of still shots or combined into a movie presentation, but do not show the entire length of a tube at any single moment. This forces a reviewer to spend time transiting the images and/or movie. Embodiments of the present invention allow a reviewer of a full scan to very rapidly diagnose a condition, providing the medical diagnostic industry both the savings of large amounts of time and money, but also provides an ability to increase a diagnostic effectiveness by spending quality time in the detail of an area of interest.

Flat bed or linear scanners utilize a line of photo sensors that are arranged in one or more rows, depending on how the system detects color. These rows of photo sensors capture images of flat objects that are either moved across the detector array in a perpendicular direction to its orientation or where the array is moved over the surface to be imaged in a similar directional manner. Such flat objects may include papers, books, photos, etc. However, these types of linear scanners are not conducive to imaging non-linear objects, such as the interior of a tube.

As will be described herein, a radial scanner may be used to image the interior of a tube. Radial scanners can include optical systems capable of utilizing one or more photosensors and a mechanical, electro-mechanical, or solid state scanning optical element. This creates a line scan or a solid state array of photosensors arranged in a format such that light from the target surface can pass through the optics, which collects it in a circular format and directs it onto the sensor array.

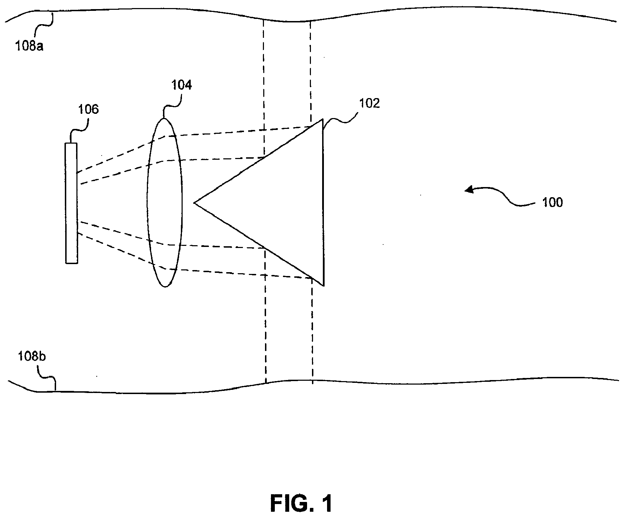

FIG. 1 is an illustration of a radial scanner system 100 according to an embodiment of the present invention. Radial scanner system 100 includes a cone-shaped mirror 102, a lens 104, and a sensor 106.

Radial scanner system 100 may be used to image the interior of a tubular structure 108. In a body, tubular structure 108 may be, for example and without limitation, part of the digestive system, such as an intestine, or part of the vascular system, such as a vein or artery. Tubular structure 108 is illustrated in FIG. 1 as a cross-section such that interior surfaces 108a and 108b are imaged by radial scanner system 100. One of skill in the art will recognize that in practice, radial scanner system 100 may image completely or partially around the interior circumference of tubular structure 108.

An image from surfaces 108a, b is reflected from cone-shaped mirror 102 towards lens 104. In an embodiment, the angle of reflection is approximately 90 degrees. Lens 104 may be, for example, a standard camera lens axially aligned to cone-mirror 102 such that light reflected from cone-shaped mirror 102 is directed onto sensor 106. Sensor 106 may be, for example, a light-detecting array, such as photosensitive pixels fabricated on a complementary metal oxide semiconductor (CMOS) substrate.

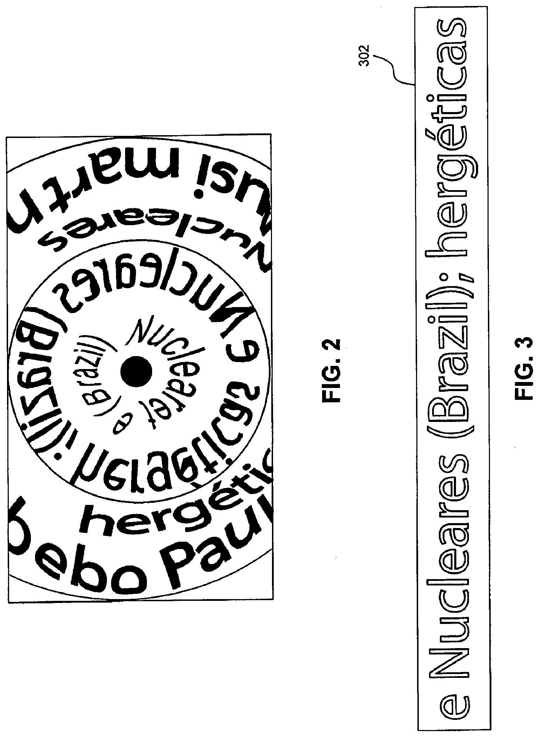

Lens 104 focuses and/or directs the image to sensor 106. Because mirror 102 is cone-shaped, the image received by sensor 106 is circular in nature. FIG. 2 illustrates an example image 202 having a circular image similar to that caused by cone-shaped mirror 102. This circularity may be removed in a post-processing software system, such that the image is uncurled into a linear dimension. FIG. 3 is an example result of processing performed on circular image 202 to create a linear image 302.

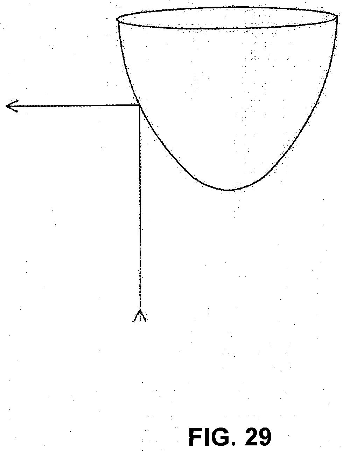

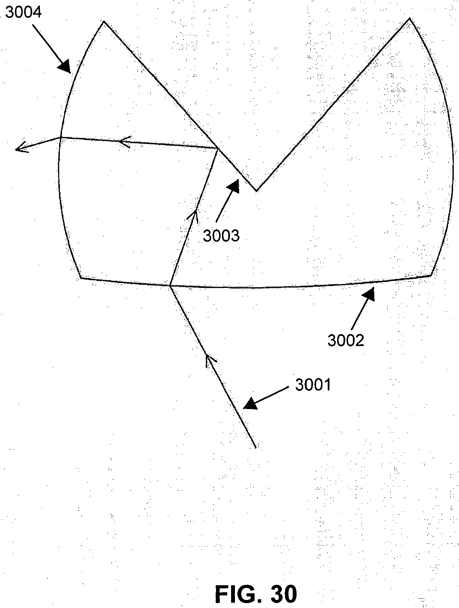

In this description, it will be obvious to one skilled in the relevant art that the cone-shaped reflective element is described for illustrative purposes only. In other embodiments, another cylindrically-symmetrical shape such as a parabola or other solid of rotation could be utilized to reflect light from the strip of imaged tissue onto the focal plane array (FIG. 29). In some embodiments, the reflective element could in fact be comprised of multiple surfaces, some of which are refractive and some of which are reflective. In FIG. 30, for example, a ray of light 3001 is first refracted (focused) at surface 3002, and then reflected from the silvered surface 3003, and finally reflected (focused) again at surface 3004. In this way, a single cylindrically-symmetrical optical element can contain both corrective (focusing) elements and the required orthogonally-reflective, cylindrically-symmetrical element needed for 360 degree scanning optics as described herein.

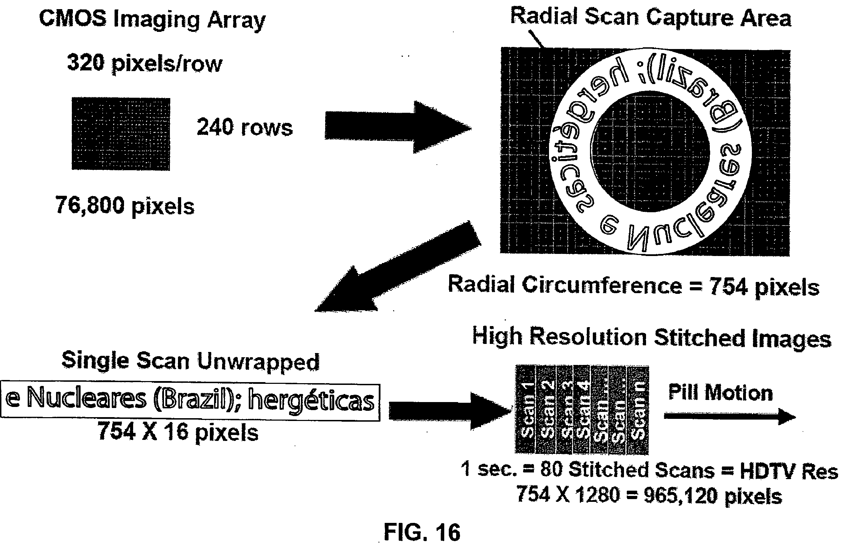

In a typical camera system, a sensor array may have, for example, 320 columns x 240 rows of pixels, resulting in a total pixel count of 76,800 pixels. In a radial scanner system, however, the image is wrapped around the circumference of a circle. If the same size array used in a typical camera is used in a radial scanner according to an embodiment of the present invention, the effective pixel count for a single layer of pixels around the circumference of the image is approximately 754 pixels. If each image has a circular line depth of, for example, 16 pixels (e.g., the number of circles of pixels in the image is 16), the effective total pixel count is approximately 754 x 16 pixels, or approximately 12,000 pixels per scan. Because the effective pixel count within a line (754 as opposed to 320) is higher than a typical camera system, the line resolution of the radial scanner system is also higher than in a typical camera system utilizing the same image sensor. Obtaining this high resolution image is further described in FIG. 16.

When imaging the interior of a tubular structure, the surface of the structure closest to the imager is of most interest to the viewer. For example, when imaging the interior of the intestines, the lining of the intestine, rather than the view down the path of the intestine, is of most interest when the viewer is searching for specific features on the intestinal wall. Additionally, the area of the image near the edge of the field of view is of higher resolution than the center portion of the field of view, since the pixel count is higher with greater image circumference. Therefore, a radial scanner system such as system 100 provides the highest resolution in the area of the image of most interest to a viewer.

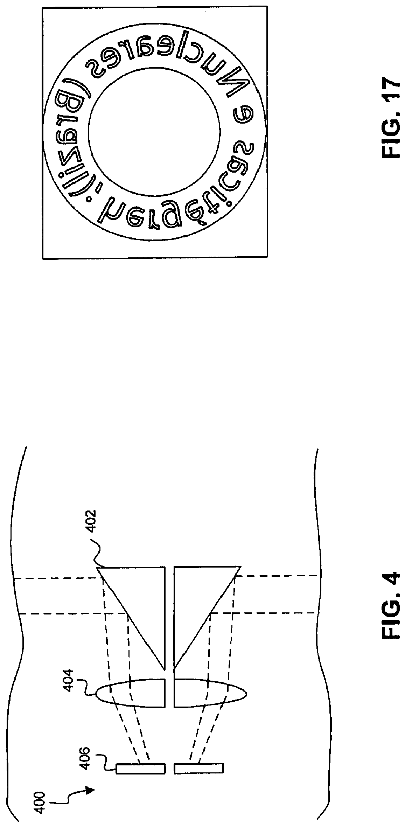

FIG. 4 is a cross-section of a radial scanning system 400 that takes advantage of this feature to provide a system requiring less power than system 100. Since the center of the field of view provided by the cone-shaped mirror (that is, the view down the tube) is of minimal interest, cone-shaped mirror 402, lens 404, and sensor 406 may be hollowed out so that only light from the edge of the field of view is imaged by sensor 406. Because sensor 406 only needs to power a ring of subsensors as opposed to a full rectangular array of subsensors, the power consumed by system 400 is reduced as compared to system 100. In an embodiment, the hollowed-out portion of the system is used for structural supports, such as spacers, as well as a pass-through for wiring or other necessary supports such as a method for illumination. FIG. 17 illustrates an unprocessed image as viewed by radial scanning system 400.

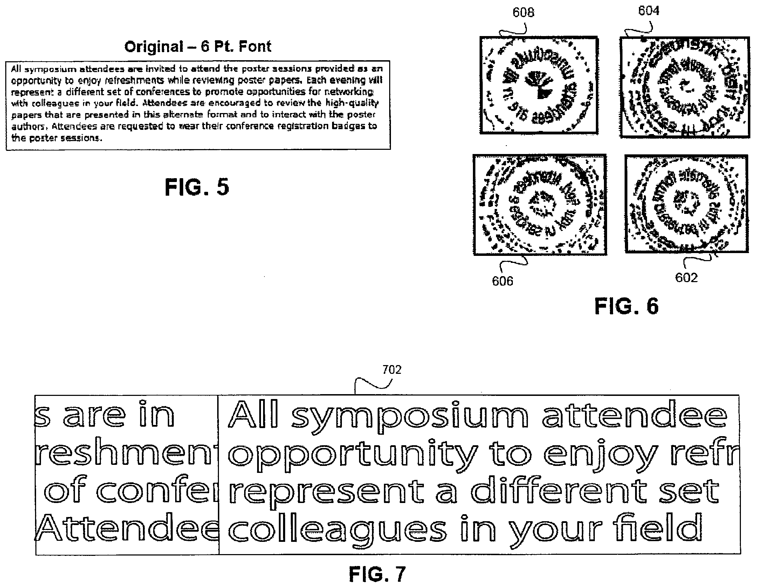

Returning to the system of FIG. 1, as radial scanner system 100 proceeds through a tube, consecutive images may be captured. Each of the consecutive images, once circularity is removed, may then be stitched together to form a full scan of the interior of the tube. FIGS. 5-7 illustrate such a process. FIG. 5 is a sample of text to be imaged. In FIG. 6, the sample of text from FIG. 5 has been coiled into a tube. Images 602, 604, 606, and 608 are resultant images from passing a radial scanning system similar to system 100 through the tube. Each of images 602, 604, 606, and 608 are taken at different locations within the tube. FIG. 7 illustrates the post-processing result of images 602, 604, 606, and 608. Each image has been processed so as to remove the circularity and produce a linear image. The multiple linear images are then stitched together to produce a scanned image 702.

The surface to be imaged may be illuminated by a light source associated with system 100. For instance, illumination may be provided by one or more light emitting diodes (LEDs). Although the illumination source will be described as including LEDs, one of skill in the art will recognize that other types of light sources, whether pulsed or constant, may also be used without departing from the spirit and scope of the present invention.

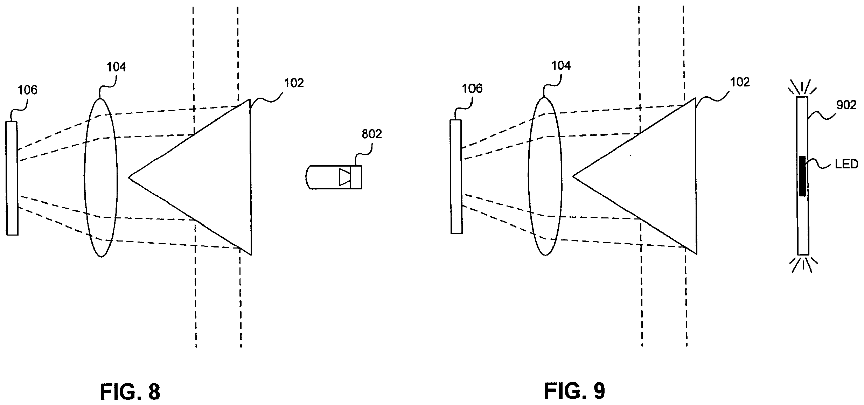

In an embodiment, one or more LEDs may be located around the edge of sensor 106. In another embodiment, one or more LEDs may be located on the side of cone-shaped mirror 102 opposite sensor 106. FIG. 8 illustrates such an embodiment. As shown in FIG. 8, positioning light source 802 behind cone-shaped mirror 102 reduces the possibility of direct light source light reaching sensor 106. In an embodiment, one or more LEDs may be placed inside an optical light dispersing ring, such that light is emitted from the illuminator in a radial strip corresponding to a section of a surface to be imaged. FIG. 9 illustrates such an optical ring 902.

If multiple light sources are used, the light sources may be of varying colors so that spectral selectivity within an image is possible. In an embodiment, red, green, and blue LEDs are used to illuminate the target surface. Sensor 106 may have different photosensing elements corresponding to the different colors so that the image is separable into spectral channels. In embodiments, very narrow bands of frequencies may be used within the general band spanned from red through blue. In additional embodiments, however, extending the spectrum into infrared and ultraviolet frequencies may provide other diagnostic data. One of skill in the art will recognize that a specific selection of light frequencies corresponding to a specific diagnostic test may differentiate several products in a family of diagnostic products based on the disclosure herein.

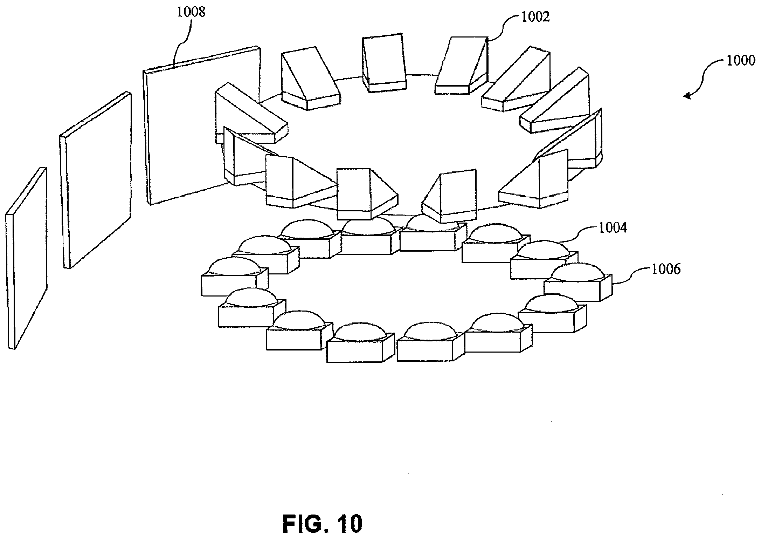

FIG. 10 illustrates a radial scanning system 1000 according to another embodiment of the present invention. Radial scanning system 1000 includes a radial set of folding mirrors or prisms 1002, a radial set of microlenses 1004, and a radial set of photosensors 1006. An image from the target surface having a field of view 1008 enters a respective folding mirror 1002. Folding mirror 1002 reflects the image onto a respective microlens 1004. In the example shown in FIG. 4, folding mirrors 1002 include prisms, in which each prism has a reflective hypotenuse surface such that light is reflected through the interior of the prism. One of skill in the art will recognize that other types of folding mirrors may be used without departing from the spirit and scope of the present invention. The corresponding microlens 1004 directs the image onto a respective photosensor 1006 to produce an electronic image of the scan region. Fields of view 1008 may overlap, such that the images produced by each photosensor 1006 corresponding to respective fields of view 1008 may be stitched together to create a continuous image. Folding mirrors 1002 are provided for a convenience of locating microlenses 1004 and photosensors 1006 conveniently on a single planar surface. However, use of folding mirrors 1002 is not necessary. In an embodiment, microlenses 1004 and photosensors 1006 may be mounted upon a flexible surface that is wrapped around itself in a circular fashion. This alternate embodiment assumes a more complicated assembly and electronic routing scheme. Although this alternate embodiment is not depicted herein, the assembly of the system would be apparent to one of skill in the art based on the disclosure provided herein.

Multi-element optical arrays 1002 and 1004 can be fabricated from single material structures, providing the ability to keep them in position. For example, radial set of folding mirrors 1002 may be created or fabricated from a single piece of material. Similarly, radial set of microlenses 1004 may be created or fabricated from a single piece of material. By utilizing alignment pins and spacers, the optical system may be easily assembled and aligned, with each element in the array kept at its appropriate location for optimal and uniform performance.

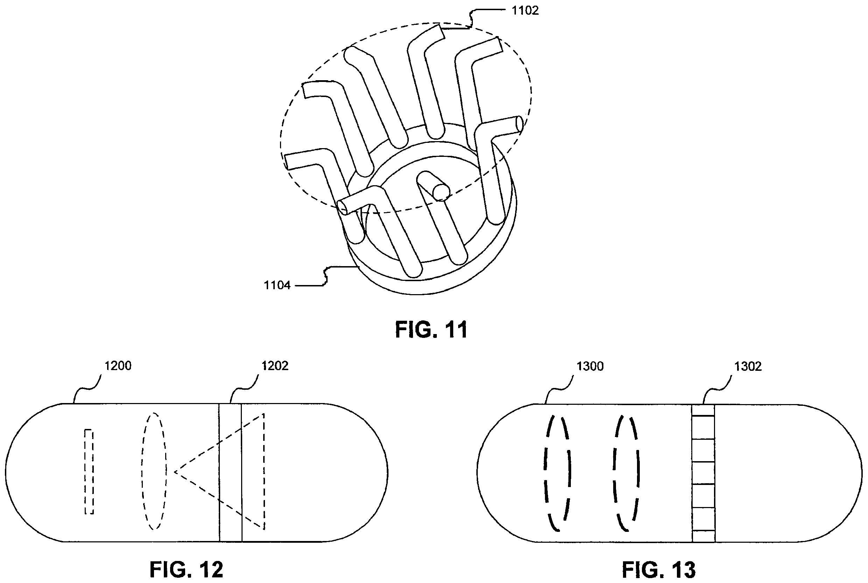

FIG. 11 illustrates a radial scanner system 1100 according to another embodiment of the present invention. Radial scanner system 1100 includes fiber optical elements 1102 connected to a ring of photosensing pixels 1104. Each of fiber optical elements 1102 may include a lens located on the tips of the fiber optical elements opposite ring of photosensing pixels 1104. In this manner, each of fiber optical elements 1102 acts as a light pipe, such that light enters through the open end of a fiber optical element 1102 and is transmitted directly to a corresponding photosensing pixel on ring 1104. Fiber optical elements 1102 may be bent at an angle needed to image an interior surface of a tube. For example, fiber optical elements 1102 may be bent such that the surface of the fiber optical element connected to ring of photosensing pixels 1104 is at approximately a 90 degree angle to the surface of the fiber optical element facing the interior of the tube. One of skill in the art will recognize that the number of fiber optical elements shown in FIG. 11 is for illustrative purposes only. Additional or fewer fiber optical elements may be used depending on one or more of: the number of photosensing pixels in ring 1104, the diameter of each fiber optical element 1102, and the amount of overlap desired between images produced by each fiber optical element. Fiber optical elements 1102 may be sized to partially or completely cover a single pixel in ring of photosensing pixels 1104. Additionally, one of skill in the art will recognize that angles less or more than 90 degrees may be used when disposing means of illumination within the same system.

Radial scanning systems according to embodiments of the invention, such as those described above, can capture multiple radial lines during any one scan to provide both color and the ability to detect and measure the motion in the axial direction perpendicular to the scan line. This ability to measure the amount of motion and direction of motion between each scan allows the scanning system to accurately stitch each scan into a continuous image of the full tubular surface. In addition, the ability to measure the motion incorporated with the known size of the pixel allows accurate measurement of the distance traveled down the tube, as well as providing accurate measurement of the size of objects observed on the tubular wall.

In one embodiment, motion is detected within an endoscopic capsule system. Motion detection within the capsule may prevent mostly or completely duplicate scans from consuming limited energy reserves without need. A motion detection device may be external to a radial scanning subsystem or included within a radial scanning subsystem.

In another embodiment, motion is detected external to an endoscopic capsule by or through a receiver worn or proximal to a patient. In this configuration, a larger (external to the body) energy reserve is available to determine motion and the extent of motion. In such an embodiment, the external system has a means for communication and control of the endoscopic capsule to deliver one or a series of scans upon the external command. In this embodiment, the endoscopic capsule can be very efficient with limited energy reserves.

Radial scanner systems such as those described above may be adapted for use in both tethered and non-tethered devices. Tethered devices include, for example and without limitation, endoscopes and medical catheters. Non-tethered devices include, for example and without limitation, an ingestible capsule or pill. Use in such devices will be described in further detail below.

FIG. 12 illustrates an ingestible capsule 1200 according to an embodiment of the present invention. Ingestible capsule 1200 contains a radial scanning system similar to that described with respect to FIG. 1. Light from surfaces around the circumference of capsule 1200 may enter the radial scanning system through aperture 1202.

FIG. 13 illustrates an ingestible capsule 1300 according to another embodiment of the present invention. Ingestible capsule 1300 contains a radial scanning system similar to that described with respect to FIG. 10. Light from surfaces around the circumference of capsule 1300 may enter the radial scanning system through an aperture 1302.

In a non-tethered device, such as capsules 1200 or 1300, images received by the photosensors may be stored on an on-board memory device and/or may be transmitted to a receiver via an on-board transmitter. An ingestible capsule having an on-board memory device and/or transmitter is further detailed in U.S. Pat. Appl. No. 11/851,221, filed September 6, 2007 and entitled, "Ingestible Low Power Sensor Device and System for Communicating with Same," and U.S. Pat. Appl. No. 11/851,214, filed September 6, 2007 and entitled, "System and Method for Acoustic Information Exchange Involving an Ingestible Low Power Capsule," each of which is incorporated by reference herein in its entirety. In other embodiments, images may be transmitted to a receiver via radio frequency (RF) emissions by typical wireless means utilizing traditional antenna and RF techniques. Furthermore, in yet another embodiment of an ingestible capsule, transmission of scanned images may occur through direct electrical connection to and conductive use of animal tissue as a communication pathway.

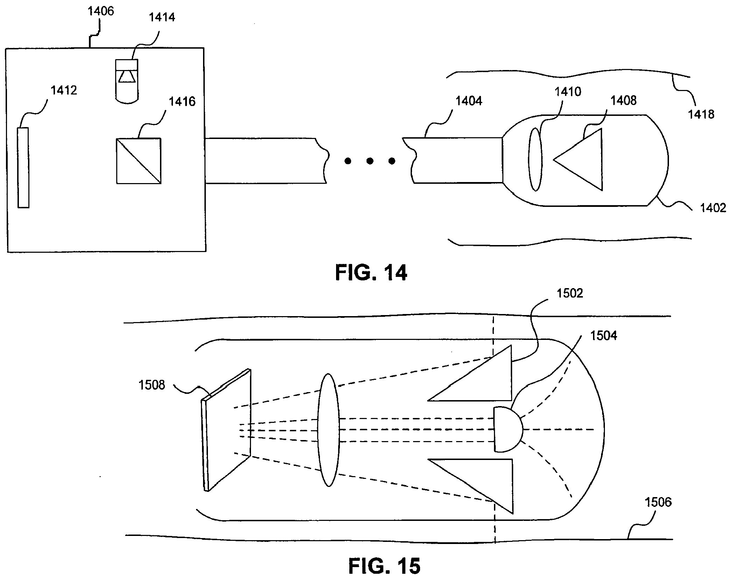

If the radial scanning system is to be used in a tethered device, components of the radial scanning system may be separated to reduce the size of an intra-cavity unit. For example, if the radial scanning system is to be used in a medical catheter, any or all of the photosensor array and the illumination source may be located external to a patient and connected to the reflector component via one or more fiber optic cables. FIG. 14 is an illustration of a tethered device 1400 according to an embodiment of the present invention. Tethered device 1400 includes an intra-cavity unit 1402 coupled to a distal end of a fiber optic cable 1404, and an extra-cavity unit 1406 coupled to a proximal end of fiber optic cable 1404. In the embodiment of FIG. 14, intra-cavity unit 1402 includes a reflector 1408 and one or more lenses 1410. Reflector 1408 may be, for example and without limitation, a cone-shaped mirror or a set of folding mirrors or prisms. In the embodiment of FIG. 14, extra-cavity unit 1406 includes a photosensor 1412, an illuminator 1414, and a beamsplitter 1416. Illumination light is transmitted from illuminator 1414 through beamsplitter 1416 and fiber optic cable 1404. The illumination light is reflected by reflector 1408 so as to illuminate a tubular surface 1418. A resulting image of tubular surface 1418 is reflected by reflector 1408 through lens 1410 and fiber optic cable 1404. The image then passes through beamsplitter 1416 and is detected by photosensor 1412. Since the power source for the radial scanning system may also be located external to the patient, a tethered device is not subject to the same power restrictions as a non-tethered device.

For example, a standard imager device, rectangular in nature, may be deployed as photosensor 1412. Use of a standard device may require inefficient use of power to collect then discard a portion of pixels not utilized. However, use of a standard imager device affords an efficiency of cost and an abundance of parts. Extra power is reasonably available in numerous methods external to a body cavity. Additionally, a tethered radial scanning device need not be concerned about limiting a rate of capturing a scan to be power efficient. Duplicate image information and the energy necessary to acquire the information can simply be discarded after post-processing.

FIG. 15 illustrates a cross-section of a combination camera and radial scanning system 1500 according to an embodiment of the present invention. As discussed above, a radial scanning system may be hollowed-out without losing significant image quality or resolution. In such an embodiment, a conventional camera may be placed in the hollowed-out portion of the radial scanning system. As illustrated in FIG. 15, combination system 1500 includes a hollowed-out cone-shaped mirror 1502 and a lens 1504. An image of tubular surface 1506 is transmitted to a photosensor 1508 as described above. An image from lens 1504, which represents the view down the tube, is transmitted to photosensor 1508 through the hollowed-out portion of cone-shaped mirror 1502. One of skill in the art will recognize that mirror 1502 and lens 1504 can be created or fabricated from a single piece of material, such as an optical quality injection molded plastic.

A combination camera and radial scanning system, such as combination system 1500, provides a user with a high-resolution image of the tubular surface as provided by the radial scanning system, as well as the context of tubular location as provided by the conventional camera. In the case of an endoscope, technicians navigate the insertion and extraction of an endoscope utilizing the view down the intestines. Combination of the camera with the radial scanner eliminates the need to turn and/or reposition the endoscope in order to view details on the side wall of the intestines.

### Advantages

Radial scanning solves many, if not all, of the performance issues associated with conventional bore scopes, endoscopes, endoscopic capsules, and the like. In a radial scanning approach, an image of the tubular structure is taken with a system that is physically oriented to travel along the axis of the tube, but whose optical system is designed to image a narrow cylindrical region of the tube's inner wall. In this manner, a series of lines capturing the circumference of the tube are captured as the scanner is moved physically down the axis of the tube. These line captures can be composed of a single pixel ring or multiple rings, such that individual scans can capture narrow bands of the wall. This allows for both capture of color images and tracking of the motion of the unit between scan captures so that the bands can be digitally overlapped to generate a seamless image of the entire length of the tubular structure. The resulting data file can then be an image that is formatted to be the circumference of the tube in one axis (taken at the pixel resolution of the scanner) and as many pixels in length as the number of pixels over the length of the tube that was scanned. Since the scanner takes its scans at 90 degrees to its axis, the resolution is uniform and constant over the region that is scanned, much like the same performance in flatbed scanners.

In an embodiment, a capsule endoscope configured with a radial scanner produces images that are processed and overlapped on a device worn by the patient or in close proximity to the patient. This embodiment affords a real time view as an endoscopic capsule is propelled by peristalsis through a gastro-intestinal tract.

In another embodiment, a capsule endoscope configured with a radial scanner produces images that are processed remotely, potentially intercontinentally, by use of the Internet or other wide area networking techology. A receiver located external to the patient forward information of the scanned images through a wireless network to be processed remotely into a full scanned product of digitally overlapped, multiple individual scans as (or after) an endoscopic capsule transits the intestinal tract.

Radial scanning improves distortion issues present in camera systems. Since all the pixels in the scan are equidistant to the photo sensing array and symmetrical along the optical axis, the distortion from the optics is not present. This allows a system design that minimizes optical distortion in the region of the optics used for imaging. Minimal refraction of the light passing through the system also allows for the system to have minimal or no distortion.

The problem of variable resolution is also improved. Since all the pixels in the scans are nearly equidistant to the surface being imaged, the resolution is nearly uniform. Also, since mechanical motion is used to scan down the length of the tube, the resolution is constant and uniform over the length of the tubular structure being scanned.

Since the distance between the radial scanner and the tubular wall being imaged is nearly constant, and the field of view of the scan is limited, the illumination is simple and uniform rather than variable. This also allows the surface area of any one scan to be minimal, which maximizes the optical collection efficiency of the system. This lowers the amount of light and the associated power necessary for illumination and scanning.

The number of useful pixels is also increased in radial scanning. Since the radial scanner is designed in the same geometrical shape as the object being imaged, there is a minimum amount of potentially wasted pixels. The pixels which may be wasted are associated with the requirement in the photo sensor array fabrication for uniform blocks of pixels to be fabricated in a square aligned geometry. This generates a few pixels in each block that will be on the periphery of the collected scans. This is estimated at being a single digit percentage of the number of pixels being used.

Dynamic range is also improved. With the ability to generate a scan scene where the resolution, angle of incidence to the surface, and the illumination are uniform, the dynamic range of the scans collected will be representative of the dynamic range of the reflective properties of the surface material. Whereas camera systems have images whose dynamic range is dominated by illumination and surface angle variations, the radial scanner performance can be optimized for the surface being imaged.

Since the range of the distance between the scanner and the tubular wall being imaged is set by their relative diameters, the required depth of focus is much less for most applications than what is required for camera-based axial imaging systems. This eliminates the need for multiple focus adjustments or the use of high value aperture stops. This results in the scanner being much more optically efficient and able to operate with significantly less illumination power than axial camera systems in the same application.

Radial scanner systems offer an improvement in F-stop performance issues over axial camera systems. Due to the nearly fixed distance between the scanner and the tubular wall being imaged, the optical system can be a low F-number system. This allows a maximization of the operational diameter of the lenses in the system, and maximizes the amount of light that can be handled by the system. As mentioned above, this allows for significant reduction of the required illumination when compared to a bore-aligned camera.

With the ability of the radial scanner to resolve the full circumference of the tube at a common resolution, the need for steerable or restricted field of view systems is eliminated. The radial scanner allows the generation of images of any format at variable resolution and covering any desired amount of the tubular surface.

### II. Ingestible scanning devices

An ingestible sensor device may be swallowed by a patient, such as a human, to diagnose one or more conditions of the patient. The ingestible sensor device may include a sensor configured to receive a stimulus inside the gastrointestinal tract of the patient, wherein the sensor is configured to output a signal having a characteristic proportional to the received stimulus. The ingestible sensor device may further include a communications module that transmits an acoustic signal modulated with the sensor output signal and a housing configured to have a size that is swallowable, wherein the housing substantially encloses the sensor and communications module. The patient using the ingestible sensor devices may be any type of animal, including a human. In addition, these same sensor devices may be temporarily implanted into patient for the purpose of continuous monitoring, such as with a several hour to several day diagnostic period at 'home' or at a professional care center. A sensor link module may be located on the surface of the patient to receive the acoustic signal output by the sensor. An example ingestible sensor is further described in U.S. Patent Appl. No. 11/851,221, filed September 6, 2007, and U.S. Patent Appl. No. 11/851,214, filed September 6, 2007, each of which is incorporated by reference herein in its entirety.

An ingestible sensor device may benefit from an optical scanner coupled to the ingestible sensor device. Although the present invention will be described with respect to an ingestible endoscopic sensor device, one of skill in the art will recognize that an optical scanner as described herein may be coupled to any type of ingestible device, or may be used on its own, without departing from the spirit and scope of the present invention. Scanned image data from the ingestible sensor device may be transmitted outside the body through use of acoustic data transmission, or using any other type of data transmission known to one of skill in the art.

Unlike typical camera pills, in an ingestible endoscopic scanner, the need for a focal plane to generate a two dimensional image can be eliminated by the use of a single axis or single element photo-sensor that is coupled with an optical scanning element or is physically scanned over the desired field of view. Whereas the image taken by a focal plane array is simultaneous over the entire field of view and must be scanned out to a memory for transmission, the single axis sensing array is capable of being read a line at a time. With the combination of a scanner, a broadband source, and a linear array, a point-by-point spectrum can be produced using diffractive optics.

The scan line images may be generated without the two dimensional circular lens and stop element used in existing ingestible cameras. In this manner, the single line array of pixels can scan a region such that in one axis, the linear line array provides the resolution while in the opposing axis a scanning system is utilized. In this manner, as will be described further below, the scanning element can be composed of any of a variety of opto-mechanical configurations composed of, for example, an oscillating mirror, a prism, a cylindrical lens, etc. This allows the achievable resolution of the scanned axis and the speed of imaging to be adjustable. Also, this scanning approach can be used with illumination or in combination with a joint illumination and imaging optical configuration producing a highly efficient optical imaging system. In embodiments, this construction may lead to the pixel area on the surface being imaged being defined by the spot size generated by the light source. Color images can be generated either by sequential scans with switching color light sources, or by using pixel rows on the photo-sensor array each with a color filter assigned to each row.

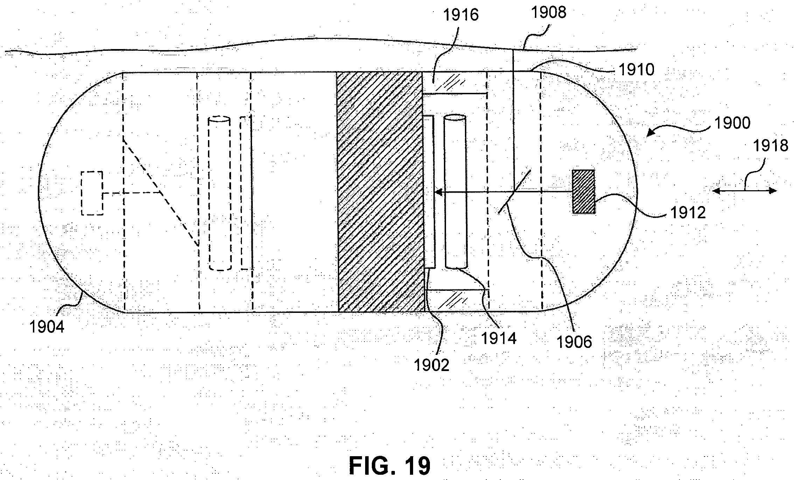

FIG. 19 illustrates an exemplary ingestible scanner 1900 according to an embodiment of the present invention. A single axis photo-sensing array 1902 is located such that it extends across the diameter of a capsule housing 1904 and is perpendicular to the axis of the capsule and the axis of housing 1904. Array 1902 may be, for example and without limitation, a single photosensing diode (also referred to herein as a photosensor or photodetector) or a one-dimensional array of multiple photosensors. Housing 1904 may be, for example, a tubular housing. A mirror 1906 within the pill is placed such that it is at an angle to array 1902 that allows array 1902 to view the intestinal wall surface 1908 through the side wall of housing 1904 through a clear aperture 1910 in housing 1904. Mirror 1906 may be, for example, an elliptical mirror. Alternatively, mirror 1906 may be a rectangular mirror that deflects on only one axis. Mirror 1906 may be placed at an angle of, for example, 90 degrees to array 1902. Mirror 1906 may be a two-sided mirror, to capture twice the data in a single revolution. Aperture 1910 may extend for 360 degrees around housing 1904. Mirror 1906 may be coupled to a micro-motor 1912 such that an electrical signal causes mirror 1906 to rotate. The axis of rotation of mirror 1906 is approximately parallel to the axis of capsule 1900. The rotation of mirror 1906 results in array 1902 scanning, in a 360 degree field, the region surrounding capsule 1900. In an alternative embodiment (not shown), mirror 1906 may be attached to a rod having two conductors and suspended in an electrical coil, such that the rod rotates as electricity is applied to the coil.

In an embodiment, a cylindrical lens 1914 is placed over array 1902 to focus light from surface 1908 reflected by mirror 1906 onto array 1902. Because array 1902 is imaging surface 1908 through aperture 1910 on the side of capsule 1900, rather than through the end of capsule 1900, resolution of array 1902 is approximately uniform at any given scan rate. Resolution may be increased in the rotational axis by adjusting the speed of rotation of mirror 1906.

Array 1902 is used to generate one or more images of surface 1908 by scanning. This is accomplished in one embodiment by moving cylindrical lens 1914, which is placed down the length of array 1902 such that the curvature of cylindrical lens 1914 is perpendicular to the length of array 1902. Scanning of the region to be imaged (such as surface 1908) is achieved by movement of the relative position of cylindrical lens 1914 to array 1902. In an embodiment, either of array 1902 or cylindrical lens 1914 can be moved. In this manner, light from different regions of the image are focused sequentially onto the pixels of array 1902 as the movement occurs. By use of cylindrical lens 1914 (or a more complex lens surface, in an embodiment) extremely wide angle images can be achieved on the axis of the image that is scanned.

In an embodiment, cylindrical lens 1914 is replaced with a scanning mirror or prism. The prism may have either a transmissive or reflective function depending upon the scanning optical design.

Illumination of the imaged wall region, such as surface 1908, may be accomplished by placing LED light sources adjacent to array 1902, to create a light distribution ring, or "light pipe" 1916. In an embodiment, light pipe 1916 evenly outputs light around approximately the entire perimeter of housing 1904. In an embodiment, light from light pipe 1916 is output at multiple frequencies. In this manner individual scans or frames of different frequencies can be captured and integrated into full color images, partial color images or false color images. Light sources may include, for example and without limitation, narrow band emitters in the visible range, white light emitters, ultraviolet emitters and infrared or near infrared emitters. Imaging arrays comprising, for example, three rows of photo-sensors, each with a specific color filter, may be used to generate full visible color images. In this manner the imaging system can be utilized to detect reflection differences in tissue over a spectral range far exceeding the abilities of the human eye. In an embodiment, a pixel size of array 1902 may be controlled through the use of mechanical shutters or a shutter wheel. The shutter wheel may include one or more color filters.

In an embodiment, light pipe 1916 is located within housing 1904. In another embodiment, light pipe 1916 is external to housing 1904. In an embodiment where light pipe 1916 is located external to housing 1904, light pipe 1916 acts to space the scanning optics off the intestinal wall. Further, positioning light pipe 1916 outside housing 1904 ensures that the light source path is separated from the received light path, with no internal reflecting surfaces.

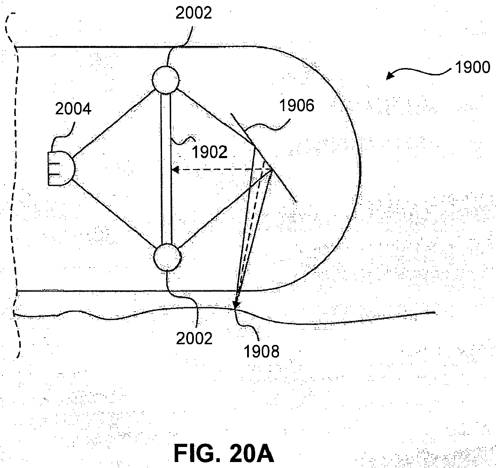

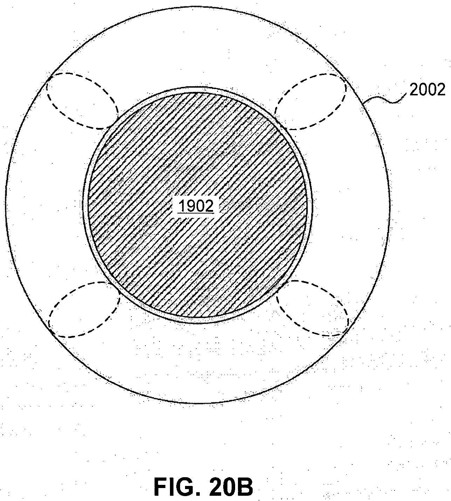

In one embodiment, the entire light pipe 1916 may be illuminated at a given time, providing illumination for the entire circumference of the intestinal wall at the same time. In another embodiment, in order to preserve power, light pipe 1916 may direct light only to the portion of surface 1908 being imaged at a given time by array 1902. FIGS. 20A and 20B illustrate an exemplary lighting system for focusing illumination light on intestinal wall surface 1908. FIG. 20A illustrates a cross-sectional view of the directional lighting system. As shown in FIG. 20A, a toroidal lens 2002 surrounds array 1902. The relationship between toroidal lens 2002 and array 1902 is further illustrated in FIG. 20B. Returning to FIG. 20A, toroidal lens 2002 directs illumination light from light source 2004 (which may be, for example, an LED) to mirror 1906. Mirror 1906 further focuses the illumination light onto surface 1908. Mirror 1906 may be positioned such that surface 1908 is approximately located at a focal point of mirror 1906. Alternatively, a collimating lens (not shown) may be included between surface 1908 and mirror 1906 to account for any focal length variability of surface 1908. An image from surface 1908 is then returned to array 1902.

Alternatively, illumination may be provided to a small portion of surface 1908 using an optical fiber to direct the illumination light accordingly. In yet another alternative, a photosensor in array 1902 may include a hole that allows light to pass through array 1902 to mirror 1906, and then be reflected onto surface 1908.

Illuminating only a small portion of the surface preserves power in the ingestible capsule, because light produced by the light source is focused onto a specific spot rather than distributed across many spots. This allows the amount of radiation output by the light source to be decreased without changing the radiation per pixel received.

Illuminating a small portion of a surface also allows image processing of the imaged surface such that very accurate colorimetry measurements may be made, according to an embodiment of the present invention. Color information may be obtained through use of an array having more than one photodetector element, such as a two- or four-element array. In one embodiment, the array may be separated into subcategories of traditional red, blue, and/or green. Alternatively, a single photodetector element may be used with multiple color light emitting diodes (LEDs), as use of a single photodetector element maximizes the sensitivity of the aperture size. This allows inspection of tissue for regions which have a slight loss of oxygen or are just becoming infected or inflamed. This technique provides higher accuracy of these conditions than that capable with white light and the human eye.

In an embodiment, the illumination intensity can be varied for different views. For example, illumination intensity may be increased when scanning points of particular interest, such as a surface of the intestinal wall. Similarly, illumination intensity may be decreased when scanning across points of lesser interest, such as down the intestine where less light will be reflected back to the detector.

Returning to FIG. 19, the scan rate, as determined by the rotational rate of mirror 1906, may be adjusted to match a desired frame rate. This requires less memory and allows more flexibility of the imaging rate. The ability to scan the image with an optical mechanical device also allows the elimination of a complex lens. Although FIG. 19 illustrates mirror 1906 as coupled to motor 1912 for rotating mirror 1906, scanning can alternatively or additionally be accomplished by moving array 1902, tilting mirror 1906 to view 360 degrees around the sides of capsule 1900, or by moving cylindrical lens 1914 to scan a region. The rate of motion of array 1902, mirror 1906, or lens 1914 defines the rate of the scan in one axis. For example, mirror 1906 may not only be rotatable around a capsule axis 1918, but it may also be tiltable, having a pivot line perpendicular to axis 1918. Such a tiltable, rotatable mirror provides a two-axis range of motion of mirror 1906.

In an embodiment, light pipe 1916 homogenizes the intensity and converts the light into a ring which illuminates the optical scanning region with a highly uniform ring of light. In a specific example, not intended to limit the present invention, mirror 1906 is oriented at 45 degrees with respect to array 1902. Motor 1912 rotates mirror 1906 and a lens is placed so that array 1902 images a 0.5 degree instantaneous field of view. The start of each scan line may be identified by the use of an occlusion in the field of view, such as wires attaching array 1902 to its supporting electronics. In this specific example, the system provides approximately 720 pixels per scan with a pixel size of 55 microns. In this example, data may be captured at 16 bits per color giving a 48 bit full color data per pixel. In this example, scan rates may vary from, for example, 1000 rpm to 10,000 rpm. This provides an example resolution of 720 x 405 pixel full color image comprising 875,000 pixels, which is approximately 1/3 the resolution of a high-definition television. In another example, the axial scan may include a full 720 pixel resolution of 720 x 1280 pixels.

In an embodiment, the scanner may use feedback from other sensors in the capsule to enter into a single color mode when a full color scan is not substantially different from a previous scan. Similarly, the scanner may enter into a multi-color mode (using two or more colors) when a single color scan is different from previous scans. In another embodiment, such color selection instructions may be received from an operator external to the capsule instead of other sensors within the capsule.

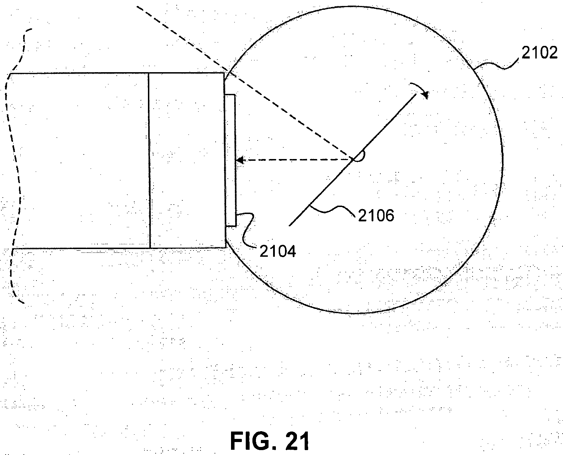

Various additional embodiments are possible using tilting and/or rotating mirrors and/or arrays. As shown in FIG. 19, a second optical system may be located on the opposite end of capsule 1900 from array 1902 and mirror 1906. This allows a 360 degree field of view for capsule 1900. FIGS. 21-25 each illustrate a different exemplary configuration for capsule 1900.

In FIG. 21, the end of capsule 2100 containing the imaging optics includes a globe-shaped housing 2102. This allows a very wide angle field to be imaged by photodetector 2104.

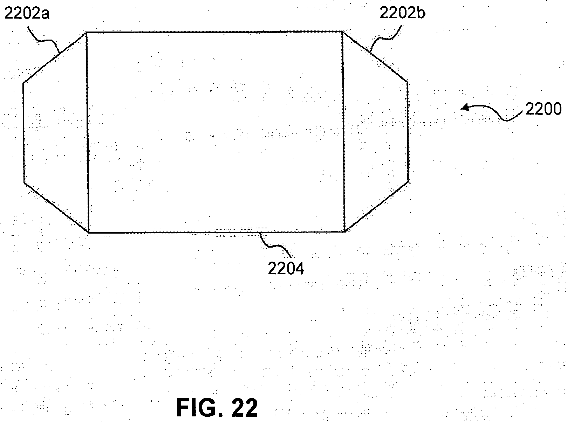

In FIG. 22, ends 2202a and 2202b of capsule 2200 are shaped like a truncated cone, so that light enters the imaging optics through a flat surface. This increases the simplicity of optics required to counteract distortion caused by light passing through housing 2204.

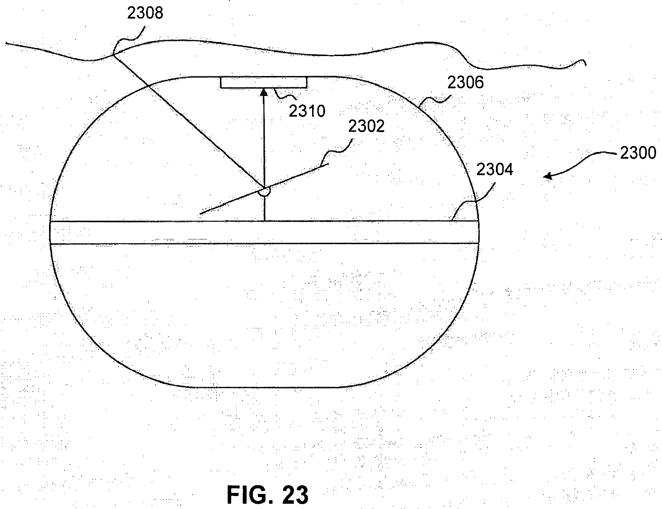

FIG. 23 illustrates a cross-section of a capsule 2300, wherein the imaging optics are not located at the ends of the capsule, but instead are located in the central portion of the capsule. Mirror 2302 is located on a central cylinder 2304. At least a portion of housing 2306 is transparent, such that an image from intestinal wall surface 2308 is reflected by mirror 2302 to photodetector 2310. Central cylinder 2304 may be rotatable, such that mirror 2302 can image around the full perimeter of capsule 2300. In an embodiment, mirror 2302 is a dish mirror to direct light to photodetector 2310 when mirror 2302 is tilted. In an embodiment, central cylinder 2304 includes an illuminator. The illuminator may be located inside central cylinder 2304 with light exiting through a slit in central cylinder 2304 (not shown) in order to illuminate intestinal wall surface 2308.

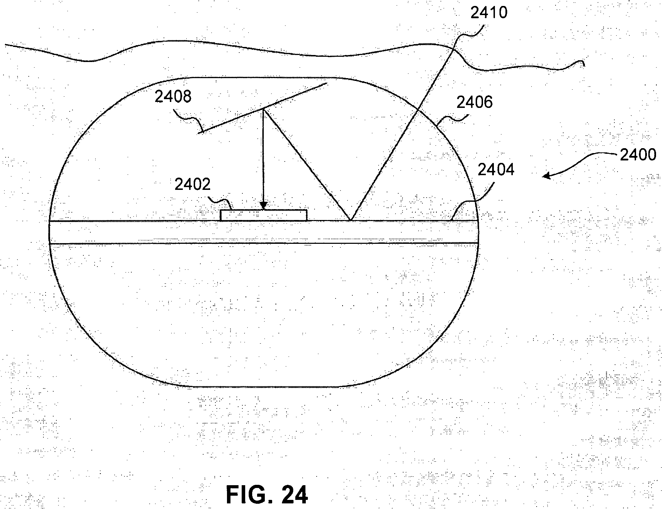

FIG. 24 illustrates a cross-section of a capsule 2400, wherein the imaging optics are located in the central portion of the capsule. In this embodiment, photodetector 2402 is located on a central cylinder 2404. Central cylinder 2404 may be made from a reflective material, such that an image from intestinal wall surface 2410 enters through transparent housing 2406 and reflects off central cylinder 2404 to a mirror 2408. The image is further reflected by mirror 2408 to photodetector 2402. Central cylinder 2404 may be rotatable, such that photodetector 2402 can image around the full perimeter of capsule 2400.

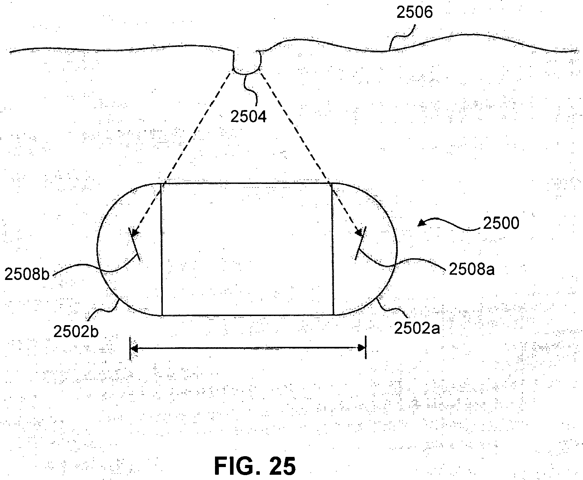

FIG. 25 illustrates a cross-section of a capsule 2500, wherein the imaging optics are surrounded by hemispherical lenses 2502a and 2502b on either end of capsule 2500. In an embodiment, hemispherical lenses 2502a and 2502b may be organized in conjunction with a pre-distorted lens to scan a respective hemisphere of the GI tract with high resolution on the side walls and graduated lower resolution toward the centerline of the scanner field of view.

In an embodiment where a 2 dimensional photodetector array is used, a conical reflector may be oriented along the center axis of the optical lens system, as illustrated in FIG. 1. In a similar embodiment, a parabolic mirror or other cylindrically-symmetrical reflector, as illustrated in FIG. 29, may be used in place of the conical reflector. In still another embodiment, this mirror element may be composed of one or more reflective and refractive surfaces, as illustrated in FIG. 30. An image may be taken of the inside of the GI tract that maximizes the number of pixels in an imaging array that present a useful image of the internal wall. In addition, illumination of the wall perpendicular to the orientation of the scanner presents a field of view with the tissues at nearly a constant distance from the scanner lens. This improves the ability to capture scan images at a uniform focus, resolution and illumination intensity.

In this embodiment, the conical reflector is designed to match the field of view of the lens and deflect the image into a circular band that is centered at or near 90 degrees to the original axis of the lens. In this manner the lens is provided with an image region where optical path lengths from one edge of the cylindrically imaged region are approximately equidistant to the path length of the opposing edge of the imaged region. A moderate field of view may thus be obtained with a normal lens. This allows simple optical elements to be used with minimal focal or distortion issues. Nearly all the pixels within the imaging array are presented with useful information. This maximizes the usable resolution in the resulting image. It is also easier to illuminate the imaged region in a near uniform manner. The images may then be processed to remove the circular distortion and produce panoramic images that represent an unwrapped and undistorted image of the interior wall.

Another embodiment whereby effects of a variable focal distance are mitigated includes changing the focus position of the lens to bring any specific distance of the imaged surface into focus. Oscillating the focus from a close position to a distant position creates a temporally focused image. This approach can then be used to determine the distance between any region of the imaged surface and the lens. By further incorporating software that can selectively capture regions of the image when they are in focus, it is possible to generate a composite image where the entire image is in focus or to generate a depth map of the surface. Once a depth map of the surface is created, additional image processing can provide a parallax calculation to be made, and images can thereby be created which represent the surface in three dimensions.

More particularly, the distance between the imaging lens and the film or image sensing array (such as a CCD or CMOS focal plane array) may vary depending upon the distance at which the imaged surface is in focus. This results in the spacing of the lens varying as various distances of the imaged surface are brought into focus. By smoothly oscillating the lens between its minimal and maximum spacing above the film or imaging array, it is possible to generate a series of images or frames where each surface region is progressively brought into focus from the closest to the farthest from the lens. Sharp objects or markings on the surfaces which represent high contrast regions (such as edges of shadows or sharp edged color differences) are sharp and high contrast only when they are in focus. The contrast of these objects drops rapidly when the lens position is changed even slightly. In this manner, it is possible to utilize software that analyzes pixel values and identifies adjacent pixels having sharp intensity variations when the lens is in other spacing positions. By capturing these regions in a sequential manner, it is possible to generate a composite image where the entire image surface is presented in sharp focus. The lens spacing information corresponding to each image may be used to create a depth profile map of the surface. This profile may then be used to generate a pseudo three dimensional image by generation of images representative of a parallax pair of images, where the position of near field surfaces is shifted more than the background surfaces.

By utilizing image processing algorithms that capture regions of an image at their highest contrast and incorporating a rapidly dynamic focusing lens system that has a narrow depth of field, the focus position may be used to generate a depth profile of the image. This depth profile may then be utilized to generate a topographic map of the imaged region. The depth profile can additionally be utilized to generate a pseudo-parallax perspective of the imaged region and present the processed image in three dimensions utilizing head-mounted stereoscopic displays or other similar devices. In addition, distortions to the image may be created to enhance the representation of depth on a single display monitor.

This allows presentation of a fully focused image of tissues which vary greatly in distance from the imaging lens, while utilizing an optical lens system designed for the highest light efficiency possible. In addition, the ability to generate depth profiles and pseudo three dimensional images can assist a physician in visualizing the relative position of the tissues, further assisting in diagnosis.

In another embodiment, a scanner having multiple photodetectors in its array enables not only spot detection but also the level of light diffused from the coherent signal sent out. The diffusion may be important, because rough tissue scatters light much more than smooth tissue. Having multiple photodetectors in the array not only offers a gain advantage in that it is more sensitive to reflected light, but it also offers an opportunity to determine a relative amount of light in the center of the image versus the outside of the image, and gives an approximate correlation to smooth tissue as opposed to rough tissue.

As has been discussed, an ingestible optical scanner may include a tiltable and/or rotatable mirror for capturing a wide angle field of view. In an embodiment of the present invention, as introduced with respect to FIG. 19, a capsule may contain two scanning systems, one on each end of the capsule. As discussed above, FIG. 25 is an illustration of an exemplary capsule 2500 having scanning optics located in two hemispherical lenses 2502a and 2502b. Depending on the field of view, it may be possible for each scanning system 2502a and 2502b to image the same feature 2504 on an intestinal wall surface 2506. Because of the effects of parallax caused by the distance d separating optics 2508a and 2508b, a three-dimensional image of feature 2504 may be obtained. Although this three-dimensional feature is described with respect to hemispherical lenses 2502a and 2502b, one of skill in the art will recognize that any type of lenses may be used without departing from the spirit and scope of the present invention.

In another embodiment, a single scanning system may image a feature such as feature 2504 from two different locations within the GI tract. In this embodiment, parallax due to the distance between the two locations may be used to provide a three-dimensional image of the feature.

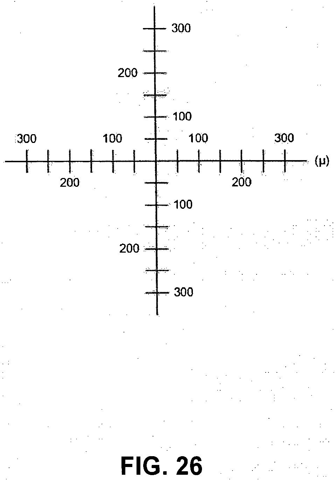

In an embodiment of the present invention, the mirror used to reflect images to the capsule's imaging sensor may have scribes located on the mirror's surface in predetermined equal distances from each other. An example set of scribes is illustrated in FIG. 26. Although FIG. 26 illustrates the distance between scribes in terms of micrometers, the distance between scribes could alternatively be fractions of mm, fractions of an inch etc. This provides accurate reference dimensions to aid a physician in identifying size of objects in view on the picture. The scribes on the mirror may result in pictures having the scribe lines always showing as a reference on each and every frame.

The mirror may also be a magnifying mirror, where the magnification is, for example and without limitation, 2x, 3x, or 4x as desired for a given purpose. This enhances the scanning capsule endoscope by increasing resolution using optical magnification. When the scribes are designed for the magnifying mirror, the magnification should be appropriately considered.

### 2. Variable resolution and/or variable magnification scanning

In an embodiment, the resolution of an axis can be controlled by the scanning optical system. This allows for higher resolution images to be generated than with existing FPA chips within the size range required of the applications. In addition, for many applications, the imaging rate (frame rate) and resolution of the images does not need to be at a fixed (e.g., video) rate. This allows the scan system to operate at variable speeds such that when triggers within the image are activated, from, for example, additional sensors within the capsule or from an operator external to the capsule, the speed of the image generation can be changed. For example, the data rate generated can be lowered when information is redundant or not of interest, and increased when specific image information is critical to the application or different from a previous scan. This increases the efficiency of the information gathering and is similar in result to image compression. In another example, a low resolution scan may be taken when the ingestible capsule is moving through the GI tract, to ensure that information regarding the portion of the GI tract through which the capsule is passing is obtained. When the capsule is not moving, the resolution can be ramped up to obtain higher resolution images of the location. In yet another example, each color may be imaged in either high resolution or low resolution depending on a previous scan and a threshold of difference from the previous scan.

Under the variable resolution scanning approach, a slower scan can be used to produce a higher resolution image on the scanning axis. The use of a cylindrical lens (such as cylindrical lens 1914) or other scanning mirror and/or prism optics provides wide angle imaging without distortion and without requiring complex optical surfaces. A radial resolution of the capsule is a function of the scan rotation rate, and is approximately equal to the line scan capture rate divided by the number of rotations per second of the scanning optics. To obtain a high resolution, for example, the capsule may capture a line scan image every degree around the viewing field. One of skill in the art will recognize that other rates of line scan image capture may be utilized without departing from the spirit and scope of the present invention. A length resolution of the capsule is a function of the linear velocity of the capsule (such as the rate at which the capsule moves through the GI tract), and is approximately equal to the number of rotations per second of the scanning optics divided by the linear velocity.

By using discrete frequency illumination, each scan can be used to collect the different reflectivity of the target surface, and therefore can be used to generate full color images and spectral analysis data at various resolutions.

Such a technique may also be used to vary the magnification of an image without any modifications to the detector. That is, if the spot size decreases while resolution stays constant, an increase in magnification results.

### 3. Photosensor construction and image data format

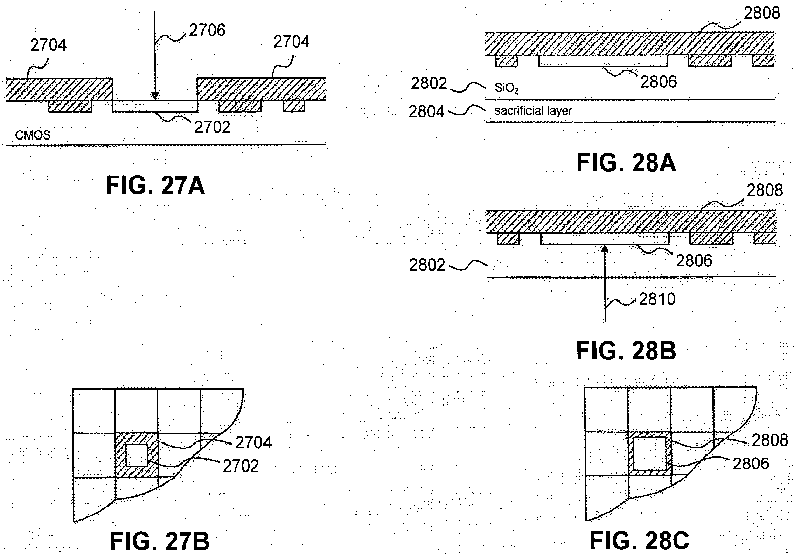

The top surface of a typical integrated circuit, including a focal plane image sensor, has nearly complete metal coverage. This metal is required to provide addressing, data readout, as well as cell, or pixel, transistor circuit connections. Each pixel's photodiode (that is, light sensor) must be placed in an area where incoming light can reach it. FIG. 27A is an illustration of a cross-section of a typical photodiode. A photosensor 2702 is implemented in CMOS. As the supporting electronics are designed depending on the characteristics of photosensor 2702, they are implemented on the chip after the implementation of photosensor 2702. Supporting electronics for photosensor 2702 are illustrated as electronics 2704. Incoming light beam 2706 enters through a hole in electronics 2704 so as to be incident on photosensor 2702. Because of the space needed for electronics 2704, the majority of the pixel area under electronics 2704 cannot be used for light sensing. This limits the available light sensitivity as well as the resolution of photosensor 2702. Dark current and coupled noise further limits the sensitivity of the photosensor 2702. This disparity between the size of photosensor 2702 and the size of a pixel containing photosensor 2702 is illustrated in FIG. 27B, which is a top-down view of a portion of an exemplary photosensor array.

Large image sensors for a given pixel density have been used to provide image resolution and light sensitivity. However, the dark current and coupled noise is a tradeoff limitation of current image sensors. Additionally, this results in a significant amount of illumination that must be supplied by LED light sources on the pill, and thus a portion of the pill's battery capacity is required for it.

In an embodiment, a Silicon-on-Insulator (SOI) CMOS image scanner may be illuminated from the back of the integrated circuit to achieve a maximum light sensitivity and finest image resolution, while enabling a small image scanner integrated circuit. FIG. 28A and 28B illustrate how such a back-lit scanner may be constructed. As shown in FIG. 28A, a base 2802 of silicon oxide (SiO₂) is implemented in place of traditional CMOS. A sacrificial metal layer 2804 is also included to provide support for base 2802. Photosensor 2806 and supporting electronics 2808 are implemented as usual, with no hole being included in electronics 2808 for accessing photosensor 2806.

As shown in FIG. 28B, once sacrificial layer 2804 is removed, a light beam 2810 may be incident on photosensor 2806 through transparent base 2802. Because the size of photosensor 2806 is no longer limited by the area requirements for supporting electronics 2808, photosensor 2806 can be made larger, as illustrated in FIG. 28C, which is a top-down view of an exemplary photosensor array. Indeed, photosensor 2806 can be made large enough to have double digit electron sensitivity or less.

This integrated circuit technology makes it possible to capture high resolution peripheral scanned images through hemispherical optics with lower image data along the center line of the optics, in accordance with an embodiment of the present invention. On-chip image scanner circuitry may also be incorporated with variable resolution in an embodiment of the present invention to trade-off resolution with the quantity of data under electronic control.

In an embodiment, optimized circuit design and layout is performed in defining the electronic arrangement of the pixel circuitry and pixel photo sensor diodes. The photosensor diodes may be substantially arranged in a circular pattern for scanning the illuminated hemispherical field of view. The hemispherical optics (such as a fisheye lens or cylindrical lens) may work in conjunction with the image scanner layout. This arrangement offers scanning information capture and output of the image in a radial data format. In an embodiment, the outer periphery of the scanning radius contains a higher density of pixels than the center, which has the lowest pixel count per unit area of silicon by simple geometry. This provides the highest resolution on the sidewall of the intestine at or near the endoscopic capsule, while lower resolution is provided down the intestinal tract. The read-out electronics may be located primarily in the center of the array where the readout lines are the shortest for low power operation. A low density of pixels may be located throughout the readout electronic region for coverage in the center of the hemispherical scanned image which looks down the intestinal tract. The four corners may also used for image electronics, since there are no pixels located there.

In an embodiment, three dimensional data is derived by combining multiple images, especially those that form opposite ends of the capsule. Post-processing of this combined data may be performed for areas of interest by the physician's desk. Once an area of interest is selected, the data may be processed and viewed at the operator's command. Three dimensional viewing modes may be similar to fly-over map viewing having controls for elevation and azimuth.

Further regarding this embodiment, the primary data format has 0 to 360 degrees around the pill plotted on the conventional x-axis and distance down the intestine plotted on the conventional y-axis. This data format may be presented on a single page so that the entire intestine can be observed quickly as a full page thumbnail. From this view, a mouse may be used to zoom in on areas of interest (e.g., from operator observation or selection of areas of interest can be computer-assisted). Higher resolution data may be zoomed in for regions of interest. In these regions, three dimensional enhancement that may be viewed in a fly-over mode employs a similar effectiveness to fly-over map controls. Location information can be presented in combination with selection of areas of interest. These and many other data presentation modes are an outcome of image scanning, as opposed to conventional movie picture imaging. Post-processing may be employed to render this data into a more conventional format of looking down the intestine so that normal data output is available.

To limit the data for lower resolution pictures and to increase the light sensitivity, groups of pixels may be combined in the low resolution mode. This pixel combination can be performed on the array or during processing external to the array. For instance, groups of four or more neighboring pixels may be combined. Similarly, image data compression may be performed by examining the neighboring pixels electronically. In a specific example not meant to limit the present invention, a low resolution 320 x 320 = 100k pixel frame may become a 640 x 640 = 400k pixel frame with a 4x magnified image resolution. In this example, a 16x image magnification is 1280 x 1280 = 1.6M pixel frame. Further according to this example, a 64x magnification renders 2560 x 2560 = 6.5M pixel resolution. Due to the image scanning technology described above, the data out before data compression is about one-fourth that of a conventional imager. The excessively high amount of data output for a full scan in the highest resolution mode may be limited by smart sensor technology in the capsule electronic controls.

In an embodiment of the present invention, electrical potentials related to peristalsis may be sensed differentially from electrodes near either end of the capsule. These electrodes may also be used to load data, electronically test, sense ingestion, and turn the capsule off or on independently. Additionally, as will be described further below, intestinal pressure waveform data can be used to determine movement of the capsule. In this manner and according to a further embodiment of the present invention, under program control the scanner may gather a low resolution image data during peristalsis and progress to stages of higher resolution scanning while the local area of the intestinal tract is quiet. The use of these high resolution modes can be used to examine parts of the intestine on the cellular level where some forms of pre-cancer have been observed.

In an embodiment, after these engineering features are implemented on the SOI CMOS semiconductor chip, completed wafers may be fabricated on the semiconductor processing line. As an additional final manufacturing step, the substrate on which the SOI wafer is constructed may be removed down to the buried Oxide (BOX). In an embodiment, this yields a cellophane-like semiconductor which may be "flip-chip" mounted on the focal plane area of a PC-board or flex circuit in the capsule.

For a full spherical image scanner, both ends of the capsule may contain one of these image scanners with its respective optics.

### 4. Arbitrary sampling scanner

Scanning systems such as facsimile machines have a specifically defined resolution coordinating to the data they acquire, which is typically defined by the spot size or pixel size of the imaging array. It is possible to sub-sample the optical pixel to acquire higher resolution. In digital systems, this is typically done by utilizing more discrete pixels than optical spots, thereby requiring a higher photodetection resolution than optical resolution. This is a costly and inefficient use of the pixels. The desire for higher resolution images with basic system designs has pushed the need for higher pixel counts for starring arrays such as focal plane array (FPA) CMOS or CCD imagers.

A scanner utilizing movement of the target or movement of the sensors provides the ability to utilize high response speeds to gain higher resolution data.

In an embodiment of the present invention, the analog response of a photodetector can be utilized to capture image data from an optical scanner so that the final resolution of the system is described by a combination of the optical spot size on the target surface being imaged and the amount of sub-sampling accomplished by an analog to digital (A/D) converter. Since A/D converters have the capability to sample at extremely high data rates, this allows the scanner to be arbitrary in resolution within the confines of the response speed of the photodetector and the illumination spot size of the scanner optics. As the scanner's illumination spot moves across the scanning range, its response can be much faster than the scanning rate. In this manner, the value of the signal from a high speed photodetector responds to changes in the intensity of the scanned spot as it moves within the diameter of the original spot. Plotting the response of the photodetector shows changes in the value of the detected signal corresponding to changes in the surface of the object that are much smaller than the optical spot size. This allows a high speed A/D converter to generate several sub-samples of the image before the spot has moved to cover a completely new surface area adjacent to the initial image spot. This sub-sampling ability allows higher resolution details in the object to be detected and imaged. Mapping of the changes in the sub-sampling data allows calculations of the position, size, and intensity of surface features significantly smaller than the optical spot size.

### 5. Video or scanned image audio content indicator

Humans observing continuous visual data become numb to sudden, short-lived, or unexpected changes in the image. This psycho-physical reaction is part of the human eye-brain response and is a known issue with monitoring security cameras as well as reviewing continuous streams of data from instruments such as medical monitoring equipment.

Because of this, reviewing long data streams of video images from optical scanning systems for medical applications is difficult, particularly when a majority of the scans have very similar data showing normal tissue, while a small selection of scans may have indications of disease or other medical issues of key interest.

In cases where video or scanned image streams have a majority of similar content and human monitoring or reviewing is tedious, auditory signals may be used as indicators of sudden change in the image content. For example, some modern security systems utilize temporal difference filtering of images to set off alarms when there are sudden changes in the scene so as to alert security guards of possible intrusions. Similarly, medical image data can be processed to generate cues to alert a physician or observer when the scans show tissue abnormalities.

In an embodiment of the present invention, the overall intensity profile of each line or frame may be determined by utilizing the intensity of each of the color channels of the scanner. When objects within the image change the parameters of these levels, the change in intensity values may exceed the normal range for the data stream. This intensity level data may be assigned an acoustic tone value which may be for the sum of the color values. In an embodiment, a tone may be assigned for each color channel being imaged to generate a set of tones. When multiple tones are used, a chord may be established to indicate data that is within a normal specification range, while data that exceeds the normal range may be assigned tone values to generate discordant tones whose assignments may be made to indicate the amount that the data exceeds the normal data range. Tone intensities may also be used to indicate optical channel intensities, range values outside of the normal data range, or the percentage of a frame and/or region where values exceed the normal. User selection may be made to eliminate the tones indicating normal values so that data exceeding the normal data range will generate alert tones. In addition, solid tones may be replaced with specific types of music or other acoustic media where subtle changes in the sound obtain the attention of the observer and alert the observer of the event in the video image or data.

In an embodiment, the imaging system may include a feed of the scanner video or data stream to a computer, where the value setting of the feed is converted into audio signals via software. The value setting may then be passed to an audio system via cables or wireless connections. This type of data processing may be accomplished with, for example and without limitation, a microcontroller or FPGA, which may be incorporated with other components within the data stream handling electronics.

In the case of patient wearable systems such as wearable monitors, this type of audio alarm may be used to notify the patient and/or physician via, for example, cell phone or wireless link, that the monitor has identified data exceeding the normal data range limits.

In this manner, the system user can be assured to be notified of the presence of the anomaly. With individual color tone generation and anomaly size to intensity generation, unique acoustic signatures may be associated with the nature of the anomalies, further providing the physician or observer with acoustic diagnostic information. Tonal shifts in the data values provides the human observer with a second sensory input to prevent missing important events in otherwise tedious data, and allows review of data at high speeds. Further, this acoustic assignment process may be used to highlight specific images in data prior to human review, allowing the data stream to be filtered to show only the images where the data has exceeded normal values.

### 6. Monitoring peristalsis

During a peristalsis contraction, a select region of the GI tract tissue is compressed by the muscle fiber contained within its structure. This compression is how the body normally moves food and waste products through the GI tract. Monitoring of peristalsis or the muscle activity with the gastric intestinal tract is critical to evaluation of the patients ability to process and move food through the body. Damage caused by disease, nerve damage, rupture or atrophy of the muscles lining the gastric intestinal tract (including the stomach) are causes of serious conditions that can be life threatening.

In an embodiment of the present invention, the ingestible endoscopic capsule can utilize peristalsis, and other muscle contractions of the GI tract, to provide data regarding the extent and nature of the contraction. Additionally, the capsule may utilize the contraction to control the functions of the pill such as powering up, transmitting data, taking images, etc.

For example, pressure sensor(s) may be used within the ingestible capsule such that these contractions are be monitored and utilized to control the timing of acoustic transmission of data and the collection of images and other sensor data. During these contractions the tissue is squeezed against the external wall of the capsule, providing the highest acoustic coupling possible and thereby the most efficient time for acoustic signals to be sent with minimal reflections from within the gastric intestinal structure. This increase in coupling allows the capsule to utilize minimal power for transmission as well as provide an enhancement in the ability to locate the position of the capsule, for example, in three dimensions from acoustic detectors placed on the patient's skin. In addition, since the capsule is not in any significant motion between contractions the continuous collection of data such as images between contractions generates data redundancy with little value to the examining physician. Therefore, the pressure provided by the contraction can also be utilized to activate the capsule's transmission system and/or initiate data collection. Along with this, images of the tissue within the GI tract that is in direct contact with the surface of the capsule provides the ability to see the tissue with minimal distortion, unlike when the tissue is relaxed and the distance from one region of the tissue is significantly different from another region within the same image.

In another embodiment of the present invention, monitoring of the activity within the gastric system is accomplished using an ingestible capsule sensor to detect electrical signals corresponding to muscular contractions associated with peristalsis. Detecting electrical emissions from nearby muscle activity and communicating the information via an acoustical link to sensors mounted on the skin of the patient allows both a detailed analysis of the peristalsis function of the gastric intestinal tract and a 3 dimensional map of the location of the pill as it collects data to be provided. This provides physicians with the location and extent of functional anomalies within this system.

The capsule peristalsis sensor may contain electrical field sensors such as those used in EKG and muscle activity sensors in other biological monitors. The capsule may process these electrical signals and use an onboard microcontroller to modulate a piezoelectric crystal also contained along with a battery power source within the capsule. As described above, the modulated acoustic signal from the capsule containing the electrical muscle activity data is then received by acoustic sensors contained within patches on the skin of the patient. These patches may be distributed across the body in such a manner as to provide a three dimensional location of the pill as it is transmitting. An exemplary method and system for locating an ingestible sensor is further described in U.S. Patent Appl. No. 11/851,179, filed September 6, 2007, which is incorporated by reference herein in its entirety. Various embodiments of this sensor approach can combine other sensors including imaging.

Once the location is known, scanned images may be combined with more traditional data to provide a more detailed understanding of the scanned images. For example, scanned images may be combined with data from a traditional magnetic resonance imaging (MRI) procedure or from a traditional ultrasound.

### III. Conclusion

While specific embodiments of the invention have been described above, it will be appreciated that the invention may be practiced otherwise than as described. For example, the invention may take the form of a computer program containing one or more sequences of machine-readable instructions describing a method as disclosed above, or a data storage medium (e.g., semiconductor memory, magnetic or optical disk) having such a computer program stored therein.

The descriptions above are intended to be illustrative, not limiting. Thus, it will be apparent to one skilled in the art that modifications may be made to the invention as described without departing from the scope of the claims set out below. It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

Embodiments of the present invention have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims and their equivalents.

## Claims

1. An ingestible scanning device, comprising: an ingestible capsule housing having a transparent window; a photo-sensing array located within the capsule housing; a mirror located within the housing and oriented to direct an image from a surface outside the transparent window to the photo-sensing array; and a light source for illuminating the surface outside the transparent window.

2. The ingestible scanning device of claim 1, wherein the mirror is rotatable and/or is a cylindrically symmetrical reflective element or is an element combining reflective and refractive surfaces.

3. The ingestible scanning device of claim 1, further comprising a cylindrical lens between the photo-sensing array and the mirror, the cylindrical lens being oriented to direct light from the mirror onto the photo-sensing array.

4. The ingestible scanning device of claim 1, further comprising a toroidal lens located around the photo-sensing array, the toroidal lens oriented to direct light from the light source onto the mirror.

5. The ingestible scanning device of claim 1, wherein the light source is located within the capsule housing or is attached to an outer surface of the capsule housing.

6. The ingestible scanning device of claim 1, wherein the light source is a light emitting diode or is a light distribution ring.

7. An ingestible scanning device, comprising:
an ingestible capsule housing having a transparent window;
a one-dimensional photo-sensing array comprising at least one photosensor located within the capsule housing;
a mirror located within the housing and oriented to direct an image from a surface outside the transparent window to the photo-sensing array;
a light source for illuminating the surface outside the transparent window,
wherein at least one of the mirror and the one-dimensional array is movable with respect to the housing; and
a cylindrical lens between the photo-sensing array and the mirror, the cylindrical lens being oriented so that its axis is parallel to the photo-sensing array to direct light from the mirror onto the photo-sensing array.

8. The ingestible scanning device of claim 7, wherein the mirror is rotatable, and/or is a cylindrically symmetrical reflective element or is an element combining reflective and refractive surfaces.

9. An ingestible scanning device, comprising:
an ingestible capsule housing having a transparent window;
a photo-sensing array located within the capsule housing;
a mirror located within the housing and oriented to direct an image from a surface outside the transparent window to the photo-sensing array;
a light source for illuminating the surface outside the transparent window; and
a toroidal lens located around the photo-sensing array, the toroidal lens oriented to direct light from the light source onto the mirror.

10. The ingestible scanning device of claim 9, wherein the light source is located within the capsule housing, and/or is attached to an outer surface of the capsule housing.

11. The ingestible scanning device of claim 9, wherein the light source is a light emitting diode, and/or is a light distribution ring.

12. The ingestible scanning device of claim 9, wherein a focus position of the image is variable, and/or wherein a rotation rate of the mirror is variable.

13. A medical catheter comprising: a fiber optic cable; an intra-cavity unit coupled to a distal end of the fiber optic cable; and an external unit coupled to a proximal end of the fiber optic cable, wherein the external unit comprises: a photo-sensing array configured to receive a scanned image, and wherein the intra-cavity unit comprises: a housing coupled to the distal end of the fiber optic cable, the housing having a transparent window; a mirror located within the housing and oriented to direct an image around a circumference of an interior surface of a tubular structure outside the transparent window to the photo-sensing array via the fiber optic cable.

14. The medical catheter of claim 13, wherein the intra-cavity unit further comprises a lens configured to direct the image from the mirror into the fiber optic cable.

15. The medical catheter of claim 13, wherein the mirror has a conical shape and/or is a radial set of prisms oriented such that reflective hypotenuse surfaces of the prisms direct images from the interior surface of the tube to the photo-sensing array.
